# EUROPEAN PATENT APPLICATION

(11) **EP 4 591 786 A1**
(43) Date of publication of application: **30.07.2025**
(21) Application number: 24153590.5
(22) Date of filing: 24.01.2024
(51) Int. Cl.: A61B 5/024, A61B 5/318, A61B 5/332, A61B 5/361, A61B 5/00

(54) **METHOD AND PATIENT MONITORING DEVICE FOR MONITORING A HEART SIGNAL OF A SUBJECT**

(71) Applicant: Koninklijke Philips N.V., 5656 AG Eindhoven (NL)
(72) Inventor: BONOMI, Alberto Giovanni, 5656AG Eindhoven (NL); EERIKÄINEN, Linda Maria, 5656AG Eindhoven (NL); TIEMANN, Christian Andreas, 5656AG Eindhoven (NL); SCHIPPER, Alphonsus Tarcisius Jozef Maria, 5656AG Eindhoven (NL)
(74) Representative: Philips Intellectual Property & Standards

(57) **Abstract**

The present invention relates to a method (200, 300) for monitoring a heart signal of a subject (110), the method (200, 300) comprising obtaining a physiological signal (132) of the subject (110), determining a heart rate signal (133) and/or an inter-beat interval signal (134) over time from the obtained physiological signal (132), analyzing the heart rate signal (133) and/or the inter-beat interval signal (134) to detect an irregularity indicating arrythmia, the detection of the irregularity comprising to determine a rate of change of the heart rate signal (133) and/or the inter-beat interval over time, analyzing the physiological signal (132) to detect a drop of quality, the detection of the drop of the quality comprising to determine a drop of an amplitude and/or a change of a morphology of the physiological signal (132), setting a signal output period for outputting one or more of the physiological signal (132), the heart rate signal (133) and the inter-beat interval signal (134) based on the detection of the irregularity and the drop of quality, and periodically outputting one or more of the physiological signal (132), the heart rate signal (133) and the inter-beat interval signal (134) according to the set signal output period.

## Description

### FIELD OF THE INVENTION

The present invention relates to a method, a patient monitoring device and a computer program for monitoring a heart signal of a subject.

### BACKGROUND OF THE INVENTION

Patient monitoring is used to monitor patients' health condition to allow for clinical intervention if health deterioration is detected (or still detected). Arrythmia for example may result in a life-threatening situation for the patient. Examples for arrythmia include atrial fibrillation, supraventricular arrythmia and atrial flutter. Atrial fibrillation (AFib) for example is the most common cardiac arrhythmia which causes the heart rhythm to be irregular. AFib is a frequent complication of a surgery. However, detection of AFib (e.g., after the surgery) is often difficult as the diagnosis requires electro-cardiogram (ECG) evidence. Even if prompt intervention by clinicians may not be mandatory, data of the heart signal has to be captured immediately as AFib may be transient (paroxsysmal). Delay in capturing ECG evidence may lower the chance of capturing the event and hence the chance for the patient to obtain an accurate diagnosis. Usually, patients at risk for AFib are urged to go to the hospital as soon as symptoms arise to be able to capture ECG evidence. However, this harbors the risk that the evidence is not captured quickly enough. For clinical follow-up decisions, it is important to capture such evidence even if the patient is, e.g., at home.

For such a purpose, wearable patient monitoring systems may be used to allow to remotely monitor the (cardiac) condition of patients, e.g., at home or at a hospital. Recent technological advancement in connectivity solutions allow these wearable sensors to transmit data directly to a cloud without the need for gateways or intermediate transmission devices such as a smartphone or home data hub.

CN 108154923 A discloses an intelligent bracelet and a bracelet cloud platform in communication connection with the intelligent bracelet. The intelligent bracelet is used for obtaining heart rates in real time and uploading the heart rates to the bracelet cloud platform at a first uploading frequency to be stored.

### SUMMARY OF THE INVENTION

It is an object of the present invention to enable a more reliable monitoring of a heart signal of a subject which may improve patients' safety.

In a first aspect of the present invention a method for monitoring a heart signal of a subject is presented, the method comprising:
obtaining a physiological signal of the subject;
determining a heart rate signal and/or an inter-beat interval signal over time from the obtained physiological signal;
analyzing the heart rate signal and/or the inter-beat interval signal to detect an irregularity indicating arrythmia, the detection of the irregularity comprising to determine a rate of change of the heart rate signal and/or the inter-beat interval signal over time;
analyzing the physiological signal to detect a drop of quality, the detection of the drop of the quality comprising to determine a drop of an amplitude and/or a change of a morphology of the physiological signal;
setting a signal output period for outputting one or more of the physiological signal, the heart rate signal and the inter-beat interval signal based on the detection of the irregularity and the drop of quality; and
periodically outputting one or more of the physiological signal, the heart rate signal and the inter-beat interval signal according to the set signal output period.

In a further aspect of the present invention a patient monitoring device for monitoring a heart signal of a subject is presented, the patient monitoring device comprising a processing unit configured to:
obtain a physiological signal of the subject;
determine a heart rate signal and/or an inter-beat interval signal over time from the obtained physiological signal;
analyze the heart rate signal and/or the inter-beat interval signal to detect an irregularity indicating arrythmia, the detection of the irregularity comprising to determine a rate of change of the heart rate signal and/or the inter-beat interval signal over time;
analyze the physiological signal to detect a drop of quality, the detection of the drop of quality comprising to determine a drop of an amplitude and/or a change of a morphology of the physiological signal;
set a signal output period for outputting one or more of the physiological signal, the heart rate signal and the inter-beat interval signal based on the detection of the irregularity and the drop of the quality; and
periodically output one or more of the physiological signal, the heart rate signal and the inter-beat interval signal according to the set signal output period.

In yet further aspects of the present invention, there are provided a corresponding computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the disclosed patient monitoring device and computer have similar and/or identical preferred embodiments as the claimed method, in particular as defined in the dependent claims and as disclosed herein.

The present invention is based on the findings that transmission protocols with long intervals between outputted data packets may help to save energy and thus enable long-term monitoring capabilities of (wearable or non-stationary) monitoring devices. However, transmission protocols with long intervals between outputted data packets may limit the ability to collect evidence of a heart signal and thus clinicians to initiate corresponding follow-up reactions compared to more frequent data transmissions. Furthermore, the heart rate alone as a criterion to detect arrhythmia is insufficient because many physiological factors can increase or decrease the heart rate without the patient suffering from an arrhythmia episode.

The present invention is thus based on the idea to look into the specific temporal pattern of a measured heart signal and the heart signal quality to identify when arrhythmic events are likely to happen or happening. The temporal pattern of heart signal and the heart signal quality is characteristic during AFib and may be used to more reliably detect arrhythmic episodes. A drop of quality of the physiological signal may result in a reduction in signal quality for the heart rate and the inter-beat interval estimations. Such a drop of quality of the physiological signal may be the result of, e.g., a reduced peripheral perfusion. The temporal pattern of a heart rate indicating, e.g., AFib, may not necessarily be related to arrythmia, but to a severe drop of quality of the heart rate and/or inter-beat interval estimation caused by arrhythmia. During arrythmia, a sensor measuring for example photoplethysmography (PPG) or ballistocardiography (BCG) signals may not be able to detect all the cardiac beats. The heart rate and/or inter-beat interval estimations may therefore become less reliable. By outputting the data more frequently, a more detailed diagnosis is enabled.

According to the present invention the sequence of heart rate or inter-beat interval is thus analyzed to determine the rate of change over time that may be abnormal and thereby due to arrythmia in conjunction with a loss of signal quality which may be a result of reduced peripheral perfusion. The present invention may allow to optimize a transmission protocol to allow for long-term monitoring of the patient and to detect irregularities in heart rate and/or inter-beat interval estimations that are due to the loss of signal quality as induced by arrythmia.

In the context of the present invention as disclosed therein, the term "heart signal" refers to a signal comprising information of the heart condition of the subject. The heart signal is, e.g., the physiological signal, the heart rate signal or the inter-beat interval signal, or parameters derived therefrom.

The term "physiological signal" refers herein to a signal characterizing a physiological status of a subject. The physiological signal may have a pulsatile waveform. For example, the physiological signal is a signal from an ECG, PPG, BCG, seismocardiogram (SCG) and/or accelerator sensor.

The term "morphology" of the physiological signal refers herein to the characteristics of the physiological signal, in particular the shape or form of the physiological signal, e.g., the shape or form of a pulsatile waveform. The morphology may comprise one or more of amplitude, width, duration, correspondence to neighboring peaks, etc.

In an embodiment, analyzing the heart rate signal and/or inter-beat interval signal comprises determining a variability and/or entropy of the heart rate signal and/or inter-beat interval signal. Therefore, variability and entropy of the heart rate signal may be used to differentiate arrythmia from a normal heart signal. For differentiating arrythmia from a normal heart signal, a statistical model such as neural network or logistic regression model may be used to identify an abnormal heart rate signal and/or inter-beat-interval signal by looking at increased variability and entropy over time. This may help a clinician to interpret the data, who usually looks for a lack of regularity in the beating pattern of the heart.

In another embodiment, the signal output period is decreased if the irregularity and the drop of quality are detected, in particular if the irregularity and the drop of the quality exceed a threshold or fulfill a criterion respectively. For example, a threshold criterion for an abnormal heart rate signal may be fulfilled, if the heart rate increases, e.g., by 40 to 100 bpm and then decreases, e.g., by 40 to 60 bpm in a time interval within, for example one minute. Thus, one or more of the physiological signal, the heart rate signal and the inter-beat interval signal may be outputted more often if the heart signal is determined to be abnormal. An analysis of the outputted signals, e.g., by a clinician in the remote health center, may be thus carried out earlier.

Further, a more frequent data output may indicate, e.g., the clinician in the remote health center, the subject and/or a user of the patient monitoring device that the subject is having arrythmia and that there may be further treatment or examination of the subject necessary. That the data output from the patient monitoring device is more frequent may also be determined by a software, e.g., in the remote health center.

In a further embodiment, the signal output period is increased if the irregularity and the drop of quality are no longer detected. Therefore, data may be outputted less frequently. Longer time intervals between the output of data may help to decrease the energy consumption and thus to save battery life of the patient monitoring device. Thus, long-term monitoring capabilities in spite of limited battery capacity of the patient monitoring device may be enabled. Preferably, the signal output period is only increased if the irregularity and the drop of quality are no longer detected for a certain time period. This may help to avoid quick switching back and forth between different signal output periods.

In one embodiment, the method further comprises receiving a command to set the signal output period to a default value and setting the signal output period accordingly. For example, the command to set the signal output period to the default value may be received from the remote health center via the data network or via a user interface of the patient monitoring device. For example, upon acknowledgement of the subject having arrythmia a clinician or a user of the patient monitoring device may send the command in order to decrease the energy consumption of the patient monitoring device and/or the amount of data received by the remote health center from the patient monitoring device via the data network. The default value may correspond to a default value before arrythmia has been detected.

In another embodiment, the method further comprises setting a size of data packets to be outputted, wherein the one or more of the physiological signal, the heart rate signal and the inter-beat interval signal is outputted in data packets according to the set size of data packets. The size of data packets to be outputted may thus be optimized to enable, e.g., a clinician in the remote health center, to make a diagnose. The size of the data packets may be adapted to the required detail of information for a diagnosis.

In a further embodiment, the method further comprises setting the size of the data packets to a larger size such that the data packets comprise more information of one or more of the physiological signal, the heart rate signal and the inter-beat interval signal, if the irregularity and the drop of the quality are detected. Therefore, more details of the physiological signal, the heart rate signal and/or the inter-beat interval signal may be outputted. Additionally, the obtained physiological signal, the determined heart rate signal and/or the determined inter-beat interval signal may be stored in a higher resolution, e.g., to comprise more information.

Further, large data packets may indicate to the clinician in the remote health center that the subject is having arrythmia and that there may be further treatment or examination of the subject necessary. That the size of data packets is larger (than before) may be determined by a software, e.g., in the remote health center.

In another embodiment, the method further comprises setting the size of the data packets to a smaller size such that the data packets comprise less information of one or more of the physiological signal, the heart rate signal and the inter-beat interval signal, if the irregularity and the drop of the quality are no longer detected. Therefore, the degree of information of the outputted (and transmitted) signals may be reduced. Reducing the amount of data to be outputted may help to decrease the energy consumption and thus to increase battery life. Preferably, the size of data packets is only adapted if the irregularity and the drop of quality is no longer detected for a certain time period. This may help to avoid quick switching back and forth between different sizes of data packets.

In another embodiment, the method further comprises receiving a command to set a size of data packets to a default value and setting the size of the data packets accordingly. For example, the command to set the signal output period to the default value may be received from the remote health center via the data network or via the user interface of the patient monitoring device. For example, upon acknowledgement of arrythmia a clinician or a user of the patient monitoring device may send the command in order to reduce energy consumption of the patient monitoring device and/or the amount of data received from the patient monitoring device. The default value may correspond to a default value before arrythmia has been detected.

In a further embodiment, the method further comprising storing more information of one or more of the physiological signal, the heart rate signal and the inter-beat interval signal if the irregularity and the drop of quality have been detected. The degree of information of the stored data may be thus adapted to allow to capture the arrythmia event and therefore to facilitate a medical diagnosis. The stored signals may be outputted at a later point in time. Additionally, the method may comprise receiving a command (e.g., from a remote health center) to store one or more of the physiological signal, the heart rate signal and the inter-beat interval signal according to a certain resolution and storing the signals accordingly.

In one embodiment, the method further comprising storing less information of one or more of the physiological signal, the heart rate signal and the inter-beat interval signal if the irregularity and the drop of quality are no longer detected. The degree of information of stored data may be thus adapted to save battery life and save memory if arrythmia is no longer detected and the information on the heart signal is thus not needed in a great degree.

In another embodiment, an alarm signal is outputted if the irregularity and the drop of quality have been detected. The risen alarm may allow for rapid follow-up reactions or decisions on further medical treatment or medical examination of the subject. The alarm may be outputted, e.g., directly to the subject, the user of the patient monitoring device or the clinician in the remote health center. For example, the patient himself may be alerted to go, e.g., to the hospital or to contact a clinician. Also, a clinician may be alerted to analyze the data.

In a further embodiment, an all-clear signal is outputted if the irregularity and the drop of quality are no longer detected. The all-clear signal may be outputted together with the physiological signal, the heart rate signal and/or the inter-beat interval signal and transmitted via the data network to the remote health center. The all-clear signal may also be outputted via the user interface of the patient monitoring device. This may indicate the clinician, the user of the patient monitoring device and/or the subject that there is no urgent need any more to capture the (terminated) arrythmia episode, e.g., with ECG. Preferably, the all-clear signal is only outputted if the irregularity and the drop of quality are no longer detected for a certain time period. This may help to avoid quick switching back and forth between the alarm signal and the all-clear signal.

### BRIEF DESCRIPTION OF THE DRAWINGS

These and other aspects of the invention will be apparent from and elucidated with reference to the embodiment(s) described hereinafter. In the following drawings
Fig. 1 shows a schematic diagram of an embodiment of a system according to the present invention.
Fig. 2 shows a schematic diagram of an embodiment of a device according to the present invention.
Fig. 3 shows a schematic diagram of an embodiment of a method according to the present invention.
Fig. 4 shows a schematic diagram of another embodiment of the method according to the present invention.
Fig. 5 shows features determined from the physiological signal according to an embodiment of the present invention.
Fig. 6 shows a result of an artificial neural network model according to an embodiment of the present invention.
Fig. 7 shows a model prediction for AFib according to an embodiment of the present invention.

### DETAILED DESCRIPTION OF EMBODIMENTS

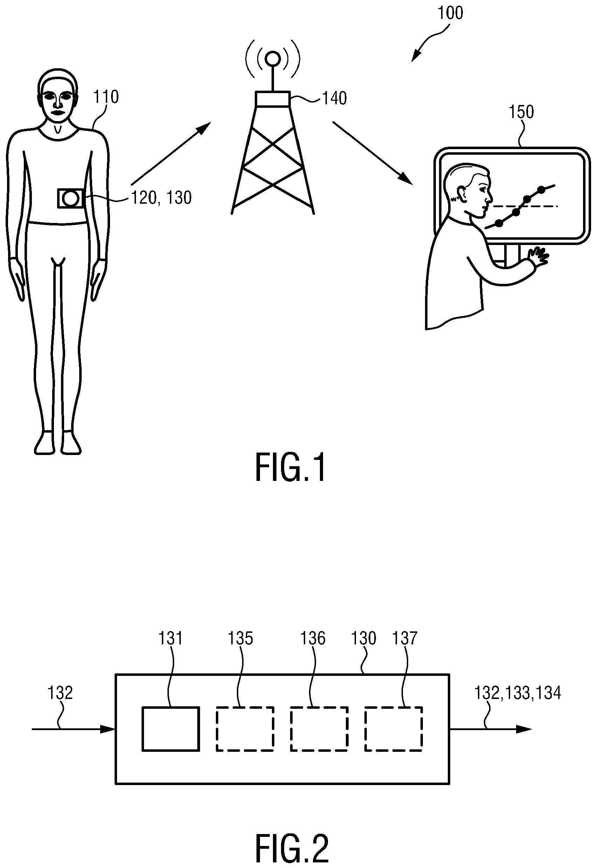

Fig. 1 shows a schematic diagram of an embodiment of a patient monitoring system 100 for monitoring a heart signal of a subject 110 according to the present invention. The patient monitoring system 100 comprises a sensor 120, a patient monitoring device 130, a data network 140 and a remote health center 150. Data measured by the sensor 120 may be obtained by the patient monitoring device 130 and outputted to be wirelessly transmitted to the data network 140. The data network 140 in turn transmits the received data to the remote health center 150, where the data may be analyzed for medical purposes. Based on the analysis of the received data, medical treatment and/or further medical examination of the subject 110 may be scheduled.

The sensor 120 is deployed on the subject 110 to measure a physiological signal relating to the physiological state of the subject 110. The sensor 120 is, for example, a BCG, PPG, ECG, SCG and/or accelerator sensor.

The patient monitoring device 130 obtains the physiological signal from the sensor 120. Subsequently, the patient monitoring device 130 determines one or more parameters from the obtained physiological signal. The physiological signal and the derived one or more parameters are then outputted and transmitted from the patient monitoring device 130 to the data network 140. To reduce energy consumption and thus to enable long-term patient monitoring for a battery-operated patient monitoring device 130, the data (i.e., the physiological signal and the one or more parameters derived therefrom) are outputted according to a settable signal output period. The signal output period may have a default value, which may be rather large, for example two hours. The patient monitoring device 130 may be a wearable patient monitoring device worn by the subject 110 such as a wearable patch or a wristband. The patient monitoring device 130 may also be a non-worn sensor such as a camera device, a bed sensor device, etc.

The data network 140 then transmits the data received from the patient monitoring device 130 to the remote health center 150. In the remote health center 150, an analysis of the data is performed, e.g. by a clinician in a hospital. The clinician can analyze the data with the help of a front-end software application and can diagnose the condition of the subject and decide on further medical examination and/or medical treatment of the subject 110. For example, the clinician may diagnose the subject 110 having arrythmia such as, e.g., AFib, and initiate stroke risk mitigation treatment.

Fig. 2 shows a schematic diagram of an embodiment of the patient monitoring device 130 for monitoring the heart signal of the subject 110. The patient monitoring device 130 comprises a processing unit 131 configured to obtain (i.e. retrieve or receive) the physiological signal 132 (from the sensor 120). From the obtained physiological signal 132, the processing unit 131 determines a heart rate signal 133 and/or an inter-beat interval signal 134. The physiological signal 132 and the heart rate signal 133 and/or inter-beat interval signal 134 are then analyzed to determine whether the subject 110 has an abnormal heart signal or a normal heart signal. Depending on the results of the performed analysis, the processing unit 131 may set an output period for outputting the physiological signal 132, the heart rate signal 133 and/or the inter-beat interval signal 134 to the data network 140.

The patient monitoring device 130 may further comprise a storage 135 configured to store the physiological signal 132, the heart rate signal 133 and/or the inter-beat interval signal 134. The patient monitoring device 130 may be configured to store the physiological signal 132, the heart rate signal 133 and/or the inter-beat interval signal 134 according to a settable degree of resolution and thus comprising more or less information.

Furthermore, the patient monitoring device 130 may further comprise an information output 136 configured to output the physiological signal 132, the heart rate signal 133 and/or the inter-beat interval signal 134 to the data network 140. The information output 136 may generally be any wireless or wired communication interface.

The patient monitoring device 130 may further comprise a user interface 137 configured to output an alarm signal to the subject 110 or a user of the patient monitoring device 130. The user interface 137 may generally be any means that outputs the alarm signal in visual, audible or haptic form, e.g. in text form, as image or diagram, as sound or spoken words, vibration, etc. For instance, the user interface may be a display, a loudspeaker or a touchscreen. For example, if according to the analysis of the patient monitoring device 130, the subject's 110 heart signal is abnormal, the user interface 137 may notify the subject 110 that medical treatment or medical examination is necessary. The notification may be also outputted to a user of the patient monitoring device 130.

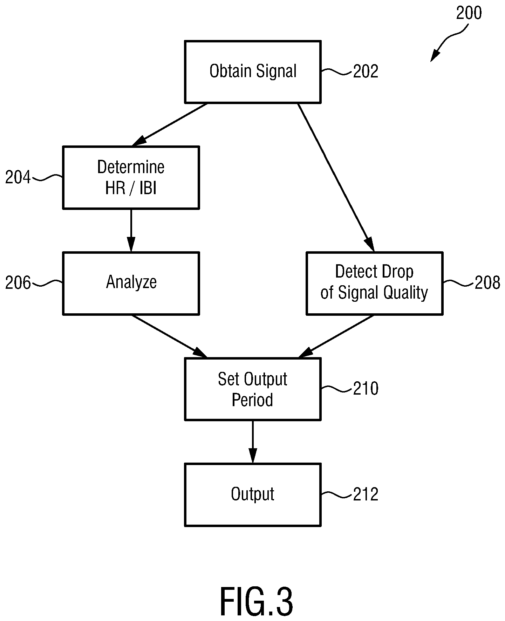

Fig. 3 shows a schematic diagram of a first embodiment of a method 200 for monitoring the heart signal of the subject 110. The steps of the method 200 may be carried out by the patient monitoring device 130, wherein the main steps of the method 200 are carried out by the processing unit 131. The method 200 may e.g. be implemented as computer program running on a computer or processor.

In step 202, the physiological signal 132 is obtained. In step 204, a heart rate signal 133 and/or an inter-beat interval signal 134 is determined from the obtained physiological signal 132. In step 206, the determined heart rate signal 133 and/or inter-beat interval signal 134 is analyzed to detect an irregularity indicating arrythmia, e.g., AFib. The irregularity may be determined by detecting a rate of change of the heart rate signal 133 and/or inter-beat interval signal 134 overtime.

In step 208, which may be carried out before, after or in parallel to step 204 and/or step 206, the physiological signal 132 is analyzed to detect a drop of quality of the obtained physiological signal 132. The drop of quality may be determined by detecting a drop of an amplitude and/or a change of a morphology of the physiological signal 132.

In step 210, a signal output period for outputting the physiological signal 132, the heart rate signal 133, the inter-beat interval signal 134 and/or one or more parameters derived therefrom is set based on the detection of the irregularity of the heart rate signal 133 and/or the inter-beat interval signal 134, and the detection of the drop of quality of the physiological signal 132. In step 212, the physiological signal 132, the heart rate signal 133 and/or the inter-beat interval signal 134 is outputted according to the set signal output period. The signals may be outputted in data packets with a default size. The size of the data packets may be settable.

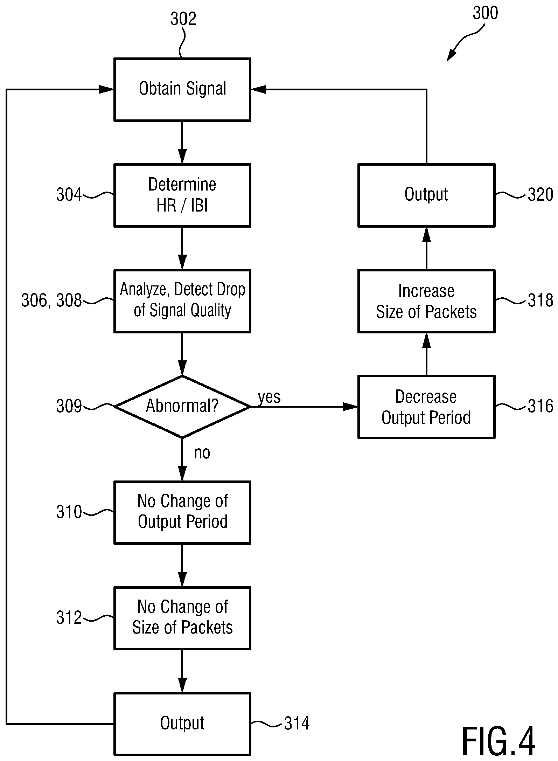

Fig. 4 shows a second embodiment of a method 300 for monitoring the heart signal of the subject 110. Steps 302 to 308 of method 300 correspond to steps 202 to 208 of method 200 (Fig. 3). The signal output period and the size of the data packets may be set to a default value, respectively (the default values differing from each other).

If, based on the analysis in step 306 and step 308, a normal heart rate signal is determined (i.e., the subject has no arrythmia such as AFib) in step 309, the signal output period is not be changed (i.e., the signal output period may remain set at the default value) or may be set to the default value in step 310. In step 312 the size of the data packets is not be changed (i.e., the size of the data packets may remain set at the default value) or may be set to a default value. In step 314, the data is outputted according to the set (default) signal output period and the set (default) size of data packets. The steps 302 to 314 may then be repeated until an abnormal heart signal is determined.

If, based on the analysis in step 306 and step 308, an abnormal heart signal is determined (i.e., the subject has arrythmia such as AFib) in step 309, the signal output period is decreased in step 316. In step 318, the size of the data packets is increased. In step 320, the data is outputted according to the set signal output period and the set size of the data packets. The patient monitoring device 130 may thus output the data (i.e., the physiological signal, the heart rate signal 133 and/or the inter-beat interval signal 134) more frequently to the data network 140 with larger data packets comprising more information.

Furthermore, the method 300 may further comprise storing the physiological signal l31, the heart rate signal 132 and/or the inter-beat interval signal 133 according to a settable resolution respectively. If an abnormal heart rate of the subject 110 is detected, the physiological signal 131, the heart rate signal 132 and/or the inter-beat interval signal 133 may be stored in a higher resolution comprising more information (e.g., each signal may be stored in a different resolution). For example, the patient monitoring device may modify the signal output period without outputting the one or more signals immediately, and at the same time storing more information of the heart signal for later output (with the newly set signal output period). If the abnormal heart signal is no longer detected, the physiological signal 131, the heart rate signal 132 and/or the inter-beat interval signal 133 may be stored in a lower resolution comprising less information.

Additionally, if an abnormal heart signal has been detected, an alarm signal may be outputted. For example, the alarm signal may be outputted to the subject 110 and/or a user of the patient monitoring device 130. The alarm signal may be outputted via the user interface 137. The alarm signal may also be outputted and transmitted via the data network 140 to the remote health center 150.

Steps 302 to 308 and 316 to 320 may then be repeated. If an abnormal heart signal is still detected, in step 316, the signal output period may remain unchanged, i.e., the signal output period may remain at a value smaller than the default value. Similarly, in step 318, the size of the data packets may remain unchanged. If however the abnormal heart signal is no longer detected, in step 310, the signal output period may be increased, e.g., to the default value. Similarly in step 312, the size of the data packets may be set to a smaller size, e.g., the default value, the smaller data packets comprising less information of the physiological signal 132, the heart rate signal 133 and/or the inter-beat interval signal 134.

The method 300 thus allows to set the signal output period and/or the size of the data packets depending on whether an abnormal or normal heart signal of the subject 110 has been detected. The output and the transmission of the data from the patient monitoring device 130 may thus be adapted to the needs of clinician to make a medical diagnosis. Referring back to Fig. 1, in case of an abnormal heart signal, with more frequently outputted data and/or larger data packets comprising more information on the heart signal of the subject 110, the clinician may use the data to observe if the patient is still suffering from the condition. With the help of additional vital signs, the clinician may additionally determine a further health deterioration such as tachycardia, a risk for pneumonia or infection, etc. of the subject 110.

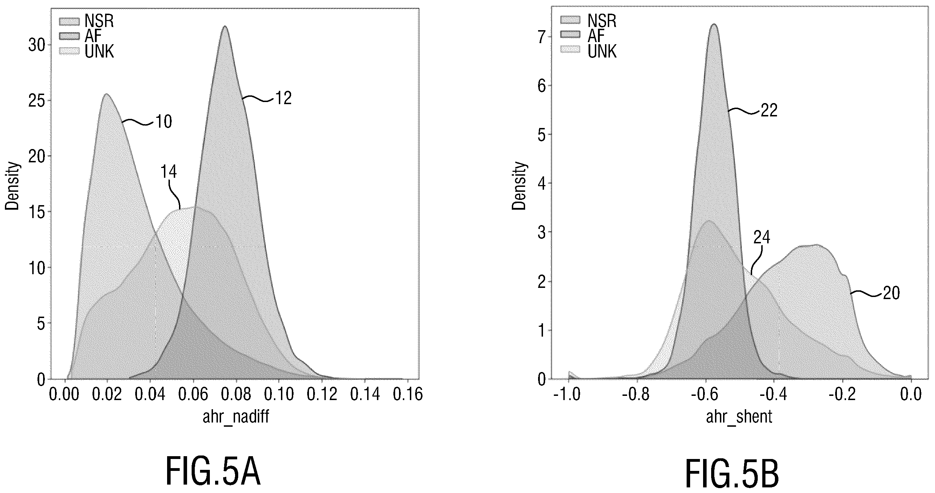

Fig. 5 shows the density over normalized differences ("nadiff"; Fig. 5A) and the density over a Shannon entropy ("shent"; Fig. 5B) which may be determined from the heart rate signal 132 and/or the inter-beat interval signal 133. The normalized differences and the Shannon entropy may be used to differentiate a normal sinus rhythm ("NSR") from a heart signal of the subject 110 having arrythmia, e.g., AFib. A threshold may be defined to differentiate between "NSR" and arrhythmia. It should be understood that normalized differences and Shannon entropy may also be determined from the physiological signal 131.

The diagram shown in Fig. 5A depicts the density over the normalized differences. Curve 10 shows a normal sinus rhythm (NSR). Curve 12 shows arrhythmia ("AF"). Curve 14 shows the unknown or undefined, when the arrythmia detection model may not determine the presence or absence of arrythmia ("UNK").

The diagram shown in Fig. 5B depicts the density over the Shannon entropy. Similar to the left diagram, curve 20 shows a normal sinus rhythm (NSR). Curve 22 shows arrhythmia ("AF"). Curve 24 shows the unknown or undefined, when the arrythmia detection model may not determine the presence or absence of arrythmia ("UNK").

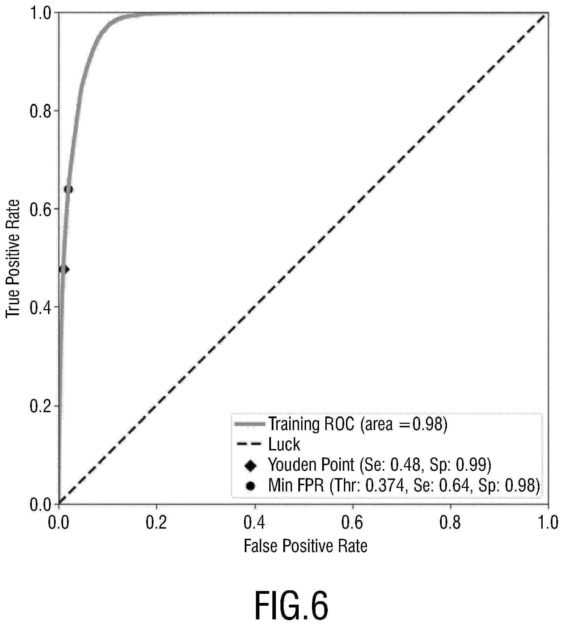

Fig. 6 shows a result of an artificial neural network model trained to differentiate a normal sinus rhythm from a heart signal characterized by arrythmia, e.g., AFib. The artificial neural network model uses normalized differences and the Shannon entropy (Fig. 5) to detect arrythmia such as AFib. Fig. 6 shows a receiver operating characteristic (ROC) curve. Black markers on the curve represent different operating points of the model that result in a different balance between True Positive Rate and False Positive Rate of arrhythmia classification.

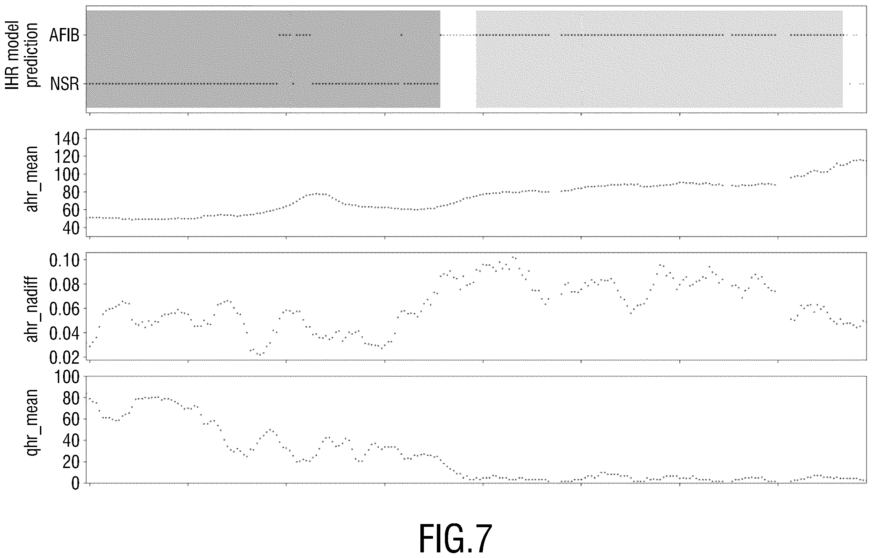

Fig. 7 shows a neural network model prediction for AFib. On the right hand side (right half of the figure), AFib has been detected by the neural network. A more frequent data output may be thus set to allow for capturing ECG documentation of AFib before the AFib episode will self-terminate. Fig. 7 shows four diagrams of different parameters over the same time period.

The first diagram shows whether arrythmia has been detected (right part) or not (left part) over time. The second diagram shows the heart rate ("ahr_mean") over time. The third diagram shows the normalized differences ("ahr_nadiff") over time. The fourth diagram shows a quality indicator ("qhr_mean") over time. From the diagrams it is visible that there is a change in the heart rate, normalized differences, and quality indicator around the time when arrhythmia is detected.

It should be understood, that while Fig. 7 has been described relating to AFib, other types of arrythmia such as supraventricular arrythmias and atrial flutter may also introduce a drop in signal quality and may be also detected by a neural network model.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Method (200, 300) for monitoring a heart signal of a subject (110), the method (200, 300) comprising:
obtaining a physiological signal (132) of the subject (110);
determining a heart rate signal (133) and/or an inter-beat interval signal (134) over time from the obtained physiological signal;
analyzing the heart rate signal (133) and/or the inter-beat interval signal (134) to detect an irregularity indicating arrythmia, the detection of the irregularity comprising to determine a rate of change of the heart rate signal (133) and/or the inter-beat interval signal (134) over time;
analyzing the physiological signal (132) to detect a drop of quality, the detection of the drop of the quality comprising to determine a drop of an amplitude and/or a change of a morphology of the physiological signal (132);
setting a signal output period for outputting one or more of the physiological signal (132), the heart rate signal (133) and the inter-beat interval signal (134) based on the detection of the irregularity and the drop of quality; and
periodically outputting one or more of the physiological signal (132), the heart rate signal (133) and the inter-beat interval signal (134) according to the set signal output period.

2. Method (200, 300) as claimed in claim 1, wherein analyzing the heart rate signal (133) and/or inter-beat interval signal (134) comprises determining a variability and/or entropy of the heart rate signal (133) and/or inter-beat interval signal (134).

3. Method (200, 300) as claimed in claim 1 or 2, wherein the signal output period is decreased if the irregularity and the drop of quality are detected, in particular if the irregularity and the drop of the quality exceed a threshold or fulfill a criterion, respectively.

4. Method (200, 300) as claimed in claim 3, wherein the signal output period is increased if the irregularity and the drop of quality are no longer detected.

5. Method (200, 300) as claimed in claim 3 or 4, further comprising receiving a command to set the signal output period to a default value and setting the signal output period accordingly.

6. Method (200, 300) as claimed in any of the preceding claims, further comprising setting a size of data packets to be outputted, and wherein the one or more of the physiological signal (132), the heart rate signal (133) and the inter-beat interval signal (134) is outputted in data packets according to the set size of data packets.

7. Method (200, 300) as claimed in claim 6, further comprising setting the size of the data packets to a larger size such that the data packets comprise more information of one or more of the physiological signal (132), the heart rate signal (133) and the inter-beat interval signal (134), if the irregularity and the drop of the quality are detected.

8. Method (200, 300) as claimed in claim 6 or 7, further comprising setting the size of the data packets to a smaller size such that the data packets comprise less information of one or more of the physiological signal (132), the heart rate signal (133) and the inter-beat interval signal (134), if the irregularity and the drop of the quality are no longer detected.

9. Method (200, 300) as claimed one of the claims 6 to 8, further comprising receiving a command to set a size of data packets to a default value and setting the size of the data packets accordingly.

10. Method (200, 300) as claimed in any of the preceding claims, further comprising storing more information of one or more of the physiological signal (132), the heart rate signal (133) and the inter-beat interval signal (134) if the irregularity and the drop of quality have been detected.

11. Method (200, 300) as claimed in claim 10, further comprising storing less information of one or more of the physiological signal (132), the heart rate signal (133) and the inter-beat interval signal (134) if the irregularity and the drop of quality are no longer detected.

12. Method (200, 300) as claimed in any of the preceding claims, further comprising outputting an alarm signal if the irregularity and the drop of quality have been detected.

13. Method (200, 300) as claimed in claim 12, further comprising outputting an all-clear signal if the irregularity and the drop of quality are no longer detected.

14. A patient monitoring device (130) for monitoring a heart signal of a subject (110) comprising a processing unit (131) configured to
obtain a physiological signal (132) of the subject (110);
determine a heart rate signal (133) and/or an inter-beat interval signal (134) over time from the obtained physiological signal (132);
analyze the heart rate signal (133) and/or the inter-beat interval signal (134) to detect an irregularity indicating arrythmia, the detection of the irregularity comprising to determine a rate of change of the heart rate signal (133) and/or the inter-beat interval signal (134) over time;
analyze the physiological signal (132) to detect a drop of quality, the detection of the drop of quality comprising to determine a drop of an amplitude and/or a change of a morphology of the physiological signal (132);
set a signal output period for outputting one or more of the physiological signal (132), the heart rate signal (133) and the inter-beat interval signal (134) based on the detection of the irregularity and the drop of the quality; and
periodically output one or more of the physiological signal (132), the heart rate signal (133) and the inter-beat interval signal (134) according to the set signal output period.

15. Computer program comprising program code means for causing a computer to carry out the steps of the method (200, 300) as claimed in any one of claims 1 to 13 when said computer program is carried out on the computer.
